# EUROPÄISCHE PATENTANMELDUNG

(11) **EP 4 723 127 A1**
(43) Veröffentlichungstag der Anmeldung: **08.04.2026**
(21) Anmeldenummer: 24204777.7
(22) Anmeldetag: 04.10.2024
(51) Int. Cl.: G16H 40/67

(54) **VORRICHTUNG ZUR ÜBERWACHUNG EINES VERTEILTEN ALARMSYSTEMS**

(71) Anmelder: B. Braun Melsungen AG, 34212 Melsungen (DE)
(72) Erfinder: SCHWARZ, Jan, 34212 Melsungen (DE); KASPARICK, Martin, 31141 Hildesheim (DE); DUESTERHUS, Michael, 37235 Hessisch Lichtenau (DE)
(74) Vertreter: Winter, Brandl - Partnerschaft mbB

(57) **Zusammenfassung**

Die Offenbarung betrifft eine Vorrichtung (10, 101) zur Überwachung eines verteilten Alarmsystems, wobei das verteilte Alarmsystem zumindest einen Server (102) und zumindest ein medizinisches Gerät (104, 105), die miteinander eine Kommunikationsverbindung (107, 108, 110) eingehen können, umfasst, umfassend: eine Erfassungseinheit (11), die eingerichtet ist, ein Bestehen einer Kommunikationsverbindung (107, 108, 110) zwischen dem Server (102) und dem medizinischen Gerät (104, 105) zu erfassen; wobei die Erfassungseinheit (11) weiter eingerichtet ist, Betriebsdaten für das medizinische Gerät (104, 105) zu erfassen; wobei die Erfassungseinheit (11) weiter eingerichtet ist, ein Abbrechen der Kommunikationsverbindung (107, 108, 110) zwischen dem Server (102) und dem medizinischen Gerät (104, 105) zu erfassen; eine Feststellungseinheit (12), die eingerichtet ist, auf Basis der erfassten Betriebsdaten festzustellen, ob das Abbrechen der Kommunikationsverbindung (107, 108, 110) entweder ein ungewolltes Abbrechen der Kommunikationsverbindung (107, 108, 110) ist oder ein gewolltes Abbrechen der Kommunikationsverbindung (107, 108, 110) ist; eine Sendeeinheit (13), die eingerichtet ist, ein erstes Signal zu senden, wobei das erste Signal ein akustisches Alarmsignal umfasst, wenn ein ungewolltes Abbrechen festgestellt wird; wobei die Sendeeinheit (13) weiter eingerichtet ist, ein zweites Signal zu senden, wobei das zweite Signal kein akustisches Alarmsignal umfasst, wenn ein gewolltes Abbrechen festgestellt wird.

## Beschreibung

### Technisches Gebiet

Die vorliegende Offenbarung betrifft eine Vorrichtung zur Überwachung eines verteilten Alarmsystems, ein computerimplementiertes Verfahren zur Überwachung eines verteilten Alarmsystems, ein Computerprogramm, ein computerlesbares Speichermedium sowie ein medizinisches Überwachungssystem.

### Technischer Hintergrund

Verteilte Alarmsysteme in medizinischen Umgebungen sind grundsätzlich bekannt. Verteilte Alarmsysteme sind Sicherheitssysteme, bei denen die Alarmfunktionen auf mehrere, geografisch getrennte oder funktionell unabhängige Komponenten verteilt sind. Diese Systeme arbeiten zusammen, um eine umfassende Sicherheitsüberwachung und -steuerung zu gewährleisten, indem sie Daten von verschiedenen Sensoren und Geräten sammeln, verarbeiten und an zentrale oder dezentrale Kontrollstellen weiterleiten.

Die Kommunikation zwischen den Komponenten erfolgt in der Regel über ein Netzwerk (z.B. Ethernet, WLAN, Mobilfunk oder andere drahtlose Protokolle wie Zigbee oder Z-Wave). Dadurch können die Alarmsysteme in Gebäuden, Städten oder auf großen Arealen eingesetzt werden.

Solche Systeme können verschiedene Arten von Alarmsystemen umfassen, unter anderen können Alarme realisiert werden, die ein Austreten eines Teilnehmers, beispielsweise eines medizinischen Geräts wie einer Infusionspumpe, aus dem verteilten Alarmsystem, insbesondere dessen Kommunikationsnetzwerk, berücksichtigen.

Die Alarme können an verschiedene Endpunkte weitergeleitet werden, beispielsweise Dashboards in Bereitschaftsräumen des Krankenhauses oder an Pager oder Smartphones der Krankenhausmitarbeiter. Die Alarme können dabei akustische und/oder visuelle Signale umfassen.

In diesem Zusammenhang hat sich herausgestellt, dass die Alarme, die bei einem gewollten Austreten aus einem verteilten Alarmsystem auftreten, als störend für die Krankenhausmitarbeiter und die Patienten wahrgenommen werden. Weiterhin hat sich herausgestellt, dass akustische Alarme Patienten verunsichern können.

### Zusammenfassung der vorliegenden Offenbarung

Es ist daher die Aufgabe der vorliegenden Offenbarung, die Nachteile aus dem Stand der Technik zu vermeiden oder wenigstens zu verringern und insbesondere eine Vorrichtung zur Überwachung eines Alarmsystems bereitzustellen, welche Patienten weniger stört bzw. verunsichert.

Die Aufgabe der vorliegenden Offenbarung wird durch eine Vorrichtung zur Überwachung eines verteilten Alarmsystems mit den Merkmalen des Patentanspruchs 1 sowie durch ein Verfahren, ein Computerprogramm, ein computerlesbares Speichermedium und ein Überwachungssystem der nebengeordneten Ansprüche 9, 13, 14 und 15 gelöst. Vorteilhafte Ausgestaltungen sind Gegenstand der Unteransprüche und/oder werden nachfolgend erläutert.

Gemäß einem ersten Aspekt der vorliegenden Offenbarung wird eine Vorrichtung zur Überwachung eines verteilten Alarmsystems bereitgestellt, wobei das verteilte Alarmsystem zumindest einen Server und zumindest ein medizinisches Gerät, die miteinander eine Kommunikationsverbindung eingehen können, umfasst, umfassend: eine Erfassungseinheit, die eingerichtet ist, ein Bestehen einer Kommunikationsverbindung zwischen dem Server und dem medizinischen Gerät zu erfassen, wobei die Erfassungseinheit weiter eingerichtet ist, Betriebsdaten für das medizinische Gerät zu erfassen, wobei die Erfassungseinheit weiter eingerichtet ist, ein Abbrechen der Kommunikationsverbindung zwischen dem Server und dem medizinischen Gerät zu erfassen; eine Feststellungseinheit, die eingerichtet ist, auf Basis der erfassten Betriebsdaten festzustellen, ob das Abbrechen der Kommunikationsverbindung entweder ein ungewolltes Abbrechen der Kommunikationsverbindung ist oder ein gewolltes Abbrechen der Kommunikationsverbindung ist; eine Sendeeinheit, die eingerichtet ist, ein erstes Signal zu senden, wobei das erste Signal ein akustisches Alarmsignal umfasst, wenn ein ungewolltes Abbrechen festgestellt wird, wobei die Sendeeinheit weiter eingerichtet ist, ein zweites Signal zu senden, wobei das zweite Signal kein akustisches Alarmsignal umfasst, wenn ein gewolltes Abbrechen festgestellt wird.

Der Begriff verteiltes Alarmsystem meint vorliegend insbesondere ein verteiltes Alarmsystem in einer medizinischen Umgebung, wie einem Krankenhaus, einer Intensivstation oder einem Pflegeheim. Bei einem verteilten Alarmsystem können medizinische Geräte über einen Server miteinander verbunden sein. Der Server kann Alarmmeldungen oder Alarmsignale von einzelnen medizinischen Geräten empfangen, diese ggfs. bearbeiten und an andere medizinische Geräte (z. B. Patientenmonitor, Infusionspumpe, Beatmungsgerät) oder andere teilnehmende Geräte (z. B. Smartphones, zentrale Überwachungsstation) weiterleiten.

Der Begriff Server meint hier insbesondere einen Kommunikationsserver in einem Netzwerk, der Kommunikationsdienste bzw. einen Datenaustausch zwischen einzelnen medizinischen Geräten und/oder teilnehmenden Geräten bereitstellt. Der Server kann hierzu Hardware und Software umfassen.

Der Begriff medizinisches Gerät meint vorliegend insbesondere eines der folgenden Geräte: Patientenmonitor, Infusionspumpe, Beatmungsgerät. Das medizinische Gerät kann vorzugweise eine Kommunikationsschnittstelle zur Kommunikation mit einem Server aufweisen. Weiterhin meint der Begriff Teilnehmer beispielsweise ein Endgerät (z.B. Smartphone, Pager) einer Pflegekraft oder ein Dashboard. Der Teilnehmer kann ein medizinisches Gerät sein. Der Teilnehmer kann eine Infusionspumpe sein. Der Teilnehmer kann ein Patientenmonitor sein. Der Teilnehmer kann ein Beatmungsgerät sein.

Der Begriff mindestens ein medizinisches Gerät meint insbesondere auch eine Vielzahl an medizinischen Geräten, beispielsweise 50 und mehr.

Der Begriff Kommunikationsverbindung meint vorliegend insbesondere einen Datenaustausch. Bei Vorliegen einer Kommunikationsverbindung kann abgeleitet werden, dass das medizinische Gerät in dem verteilten Alarmsystem angemeldet ist.

Der Begriff Erfassungseinheit meint vorliegend insbesondere eine Recheneinheit, die eingerichtet ist, das Vorhandensein einer Kommunikationsverbindung zwischen einem medizinischen Gerät und einem Server zu erfassen. Hierzu kann die Recheneinheit beispielsweise einen Heart Beat oder einen Handshake im Rahmen der Kommunikation zwischen dem medizinischen Gerät und dem Server auswerten. Die Erfassungseinheit kann vorliegend insbesondere weiter eingerichtet sein, ein Abbrechen der Kommunikationsverbindung zu erfassen. Hierzu kann sie wiederum einen Heart Beat oder einen Handshake im Rahmen der Kommunikation zwischen dem medizinischen Gerät und dem Server auswerten. Die Erfassungseinheit kann vorliegend insbesondere weiter ausgebildet sein, Betriebsdaten für das medizinische Gerät zu erfassen. Hierzu kann die Erfassungseinheit eine Datenschnittstelle aufweisen, über die sie die Betriebsdaten empfangen kann.

Der Begriff Betriebsdaten ist vorliegend breit zu verstehen und meint insbesondere Daten bzw. Informationen, die Aufschluss über ein gewolltes oder ungewolltes Abbrechen der Kommunikationsverbindung zwischen dem medizinischen Gerät und dem Server liefern. Die Betriebsdaten können dabei bei einem Entfernen eines medizinischen Gerätes aus einem übergeordneten System erzeugt werden. Beispielsweise kann das medizinische Gerät eine Infusionspumpe sein, die in einem Rack bzw. Ordnungssystem angeordnet ist. Das Herausziehen der Infusionspumpe aus dem Rack bzw. dem Ordnungssystem kann beispielsweise über Sensoren (z.B. Magnetsensor, Hallsensor) und/oder eine Datenschnittstelle (z.B. Infrarot, NearFieldCommunication, Ethernet, serielle Verbindung) zwischen Infusionspumpe und Ordnungssystem erfasst werden. Diese Information kann wieder genutzt werden, indem man ein künftiges Abbrechen der Kommunikationsverbindung als gewolltes Abbrechen der Kommunikationsverbindung einstuft. Beispielsweise kann zunächst die Infusionspumpe aus dem Ordnungssystem entnommen werden. Dabei wird über einen Magnetsensor, der beispielsweise in der Infusionspumpe verbaut ist, die Entnahme detektiert. Die Infusionspumpe sendet diese Information dann an die Erfassungseinheit der Vorrichtung zur Überwachung des verteilten Alarmsystems. Weiterhin kann eine Kommunikationsverbindung zwischen dem medizinischen Gerät und dem Server auch gezielt durch einen Anwender abgebrochen werden. Hierzu kann der Anwender beispielsweise die Aktion am Server auslösen. Hierzu wird die Information über den gezielten Abbruch dann der Erfassungseinheit über eine Datenschnittstelle zur Verfügung gestellt. Betriebsdaten können in diesem Zusammenhang beispielsweise weiterhin Daten umfassen, die Aufschluss über eine temporäre gewollte Unterbrechung einer Kommunikationsverbindung liefern. Beispiele hierfür sind u.a. Softwareupdates und Wartungsarbeiten.

Der Begriff Feststellungseinheit meint vorliegend insbesondere eine Recheneinheit, die eingerichtet ist, die Betriebsdaten auszuwerten und festzustellen, ob ein gewollter Abbruch der Kommunikationsverbindung vorliegt oder ein ungewollter Abbruch. Hierzu kann die Feststellungseinheit beispielsweise die erfassten Betriebsdaten mit vorher festgelegten Anwendungsfällen vergleichen. Beispielsweise, wenn eine Infusionspumpe aus einem Ordnungssystem entnommen wird, wird eine entsprechende Information an die Erfassungseinheit gesendet. Die Feststellungseinheit kann diese Information dann beispielsweise mit einer in einer Tabelle hinterlegten Information abgleichen. Bei einem positiven Vergleich, kann sie nun von einem gewollten Abbrechen der Kommunikationsverbindung ausgehen.

Der Begriff Sendeeinheit meint vorliegend eine Einheit, die eingerichtet ist, ein erstes und/oder zweites Signal an die medizinischen Geräte und/oder teilnehmenden Geräte des verteilten Alarmsystems zu senden.

Der Begriff Signal meint insbesondere eine Meldung. Die Meldung kann dabei eine Information umfassen. Die Information kann dabei über eine Zustandsänderung des medizinischen Geräts informieren. Die Zustandsänderung kann dabei darauf hinweisen, dass das medizinische Gerät gewollt aus dem verteilten Alarmsystem entfernt wurde. Die Zustandsänderung kann dabei darauf hinweisen, dass das medizinische Gerät ungewollt aus dem verteilten Alarmsystem entfernt wurde. Das Signal kann ein akustisches Warnsignal umfassen. Das Signal kann eine visuelle Information umfassen. Beispielsweise kann die visuelle Information eine Textnachricht, ein Anzeigeelement, ein Einblenden eines Anzeigeelements, ein Ausblenden eines Anzeigeelements umfassen. Die visuelle Information kann jeweils an einem medizinischen Gerät (Infusionspumpe) und/oder einem teilnehmenden Gerät (Smartphone) angezeigt werden.

Der Offenbarung liegt die Erkenntnis zugrunde, dass bei verteilten Alarmsystemen aktuell beim Verlassen eines Teilnehmers (z. B. medizinisches Gerät) des verteilten Alarmsystems ein akustisches Warnsignal z. B. an dem medizinischen Gerät und/oder einem mobilen Endgerät einer Pflegekraft ausgegeben wird. Aktuelle Alarmsysteme differenzieren jedoch nicht zwischen gewolltem Verlassen des Alarmsystems und ungewolltem Verlassen des Alarmsystems. Bei gewolltem bzw. planmäßigen Verlassen des Alarmsystems durch ein medizinisches Gerät bedarf es keines akustischen Alarmsignals. Dieses ist dann unnötig und stört sowohl das medizinische Personal wie auch die Patienten, bzw. verunsichert die Patienten. Die Offenbarung schlägt hier vor, auf Basis von Betriebsdaten ein gewolltes Unterbrechen der Kommunikationsverbindung zwischen medizinischen Gerät und Server zu erkennen und in diesem Fall kein akustisches Warnsignal an Teilnehmer des verteilten Alarmsystems zu senden bzw. auszugeben. Im Falle einer Erfassung eines ungewollten Abbrechens der Kommunikationsverbindung hingegen sendet die Vorrichtung ein akustisches Alarmsignal. Auf diese Weise kann bei gleichbleibender Sicherheit die Störung von medizinischen Personal und Patienten reduziert werden. Beispielsweise sind in Intensivstationen mehrere Patienten untergebracht, die wiederum an eine Vielzahl an medizinischen Geräten, wie zum Beispiel Infusionspumpen angeschlossen sein können. Hier kann es beispielsweise bei Wartungsarbeiten bzw. Softwareupdates zum gleichzeitigen gewollten Abbrechen der Kommunikationsverbindungen der Vielzahl an Infusionspumpen mit dem Server kommen. Die Erfindung kann in solchen Fällen besonders vorteilhaft den Lärmpegel reduzieren, indem sie einen unnötigen Massenalarm verhindert. Insbesondere bei einer Vielzahl an medizinischen Geräten kann dies zu einer erheblichen Lärmreduktion führen. Dies kann sich auf die Effizienz des Alarmsystems sowie die Effizienz des medizinischen Personals positiv auswirken. Gemäß einer bevorzugten Ausführungsform können die Betriebsdaten auf eine oder mehrere der folgenden Situationen hindeuten: physische Trennung medizinisches Gerät aus verteiltem Alarmsystem, Softwareupdate, Wartung medizinisches Gerät, Wartung Teilnehmer, Wartung Server, Wartung Gateway.

Der Begriff physische Trennung aus dem verteilten Alarmsystem meint vorliegend insbesondere, dass das medizinische Gerät gewollt aus dem verteilten Alarmsystem entfernt wird. Hierzu kann beispielsweise wie oben beschrieben ein medizinisches Gerät, wie eine Infusionspumpe, aus einem Ordnungssystem entfernt werden. Die Entnahme kann dabei über einen Sensor (z.B. Magnetsensor, Hallsensor) erfasst werden. Die Entnahmeinformation kann dann der Erfassungseinheit mitgeteilt werden, sodass die Vorrichtung kein akustisches Warnsignal bei Abbrechen der Kommunikationsverbindung zwischen medizinischem Gerät und Server auslöst. Weiterhin kann darunter ein bewusstes Herausnehmen des medizinischen Geräts aus dem verteilten Alarmsystem verstanden werden. Hierbei kann ein Anwender beispielsweise die Kommunikationsverbindung vom Server aus mit dem medizinischen Gerät unterbrechen und das medizinisches Gerät aus dem verteilten Alarmsystem entfernen. Diese Information kann dann wiederum der Erfassungseinheit mitgeteilt werden, sodass die Vorrichtung kein akustisches Warnsignal bei Abbrechen der Kommunikationsverbindung zwischen medizinischem Gerät und Server auslöst. Bei einem Softwareupdate kann die Information über das anstehende Softwareupdate der Erfassungseinheit mitgeteilt werden, sodass die Vorrichtung kein akustisches Warnsignal bei Abbrechen der Kommunikationsverbindung durch das Softwareupdate zwischen dem medizinischen Gerät und dem Server auslöst. Gleiches gilt für geplante Wartungsarbeiten an der Infrastruktur des verteilten Alarmsystems. Die Infrastruktur kann dabei Netzwerkkomponenten und Betriebssystemupdates umfassen. Die Wartungsarbeiten können zu einem Ausfall der Kommunikationsverbindung zwischen dem medizinischen Gerät und dem Server führen. Die Information über geplante Wartungsarbeiten kann der Erfassungseinheit mitgeteilt werden, sodass die Vorrichtung kein akustisches Warnsignal bei Abbrechen der Kommunikationsverbindung durch das Softwareupdate zwischen dem medizinischen Gerät und dem Server auslöst. Ein anderer Anwendungsfall kann eine Wartung an dem Gateway des Servers sein. Beispielsweise kann ein Zertifikatsaustausch an dem Gateway durchgeführt werden. Durch den Zertifikatsaustausch kann das Gateway kurzzeitig nicht verfügbar sein. Die medizinischen Geräte und/oder Teilnehmer verlassen dann kurzzeitig das verteilte Alarmsystem. Die Information über geplante Wartungsarbeiten an dem Gateway kann der Erfassungseinheit mitgeteilt werden, sodass die Vorrichtung kein akustisches Warnsignal bei Abbrechen der Kommunikationsverbindung durch die Wartungsarbeiten zwischen medizinischem Gerät und Server auslöst.

Gemäß einer bevorzugten Ausführungsform kann der Server ein Gateway zur Konvertierung von Datenprotokollen umfassen.

Durch die Verwendung eines Gateways können unterschiedliche medizinische Geräte, die unterschiedliche Datenprotokolle verwenden, miteinander kommunizieren.

Auf diese Weise kann vorteilhafterweise die Reichweite der Vorrichtung in Bezug auf unterschiedliche Hersteller bzw. Teilnehmer bzw. medizinische Geräte mit unterschiedlichen Datenprotokollen erweitert werden.

Gemäß einer bevorzugten Ausführungsform können das erste und/oder zweite Signal ein visuelles Signal umfassen.

Das visuelle Signal umfasst vorzugsweise ein Anzeigeelement, das ein Vorliegen einer Kommunikationsverbindung oder kein Vorliegen einer Kommunikationsverbindung umfasst. Das visuelle Signal kann weiterhin eine Textnachricht umfassen.

Gemäß einer bevorzugten Ausführungsform kann das Alarmsystem eine Vielzahl an medizinischen Geräten und/oder anderen Teilnehmern umfassen.

Das medizinische Gerät kann eines oder mehrere der folgenden umfassen: Beatmungsgerät, Patientenmonitor, Infusionspumpe.

Gemäß einer bevorzugten Ausführungsform kann das medizinische Gerät eine Infusionspumpe sein.

Gemäß einer bevorzugten Ausführungsform kann die Vorrichtung weiter eingerichtet sein, ein automatisches Aufbauen einer erneuten Kommunikationsverbindung zwischen dem zumindest einem medizinischen Gerät und dem Server nach dem erfassten gewollten Abbrechen der Kommunikationsverbindung, insbesondere nach einem Softwareupdate, durchzuführen, um in das verteilte Alarmsystem zurückzukehren.

Mit anderen Worten ausgedrückt, kann die Vorrichtung ein Kommunikationsprotokoll bereitstellen, das nach einem gewollten Abbrechen der Kommunikationsverbindung, beispielsweise einem Softwareupdate oder einer Wartung, ein erneutes Aufbauen einer Kommunikationsverbindung zwischen dem medizinischen Gerät und dem Server ermöglicht. Hierzu ist dann vorteilhafterweise kein Zutun eines Anwenders notwendig. Dies kann sich vorteilhaft auf die Effizienz des verteilten Alarmsystems auswirken.

Gemäß einer bevorzugten Ausführungsform können das erste und/oder zweite Signal an den Server zur Weiterleitung an weitere medizinische Geräte und/oder Teilnehmer versendet werden.

Mit anderen Worten ausgedrückt werden die ersten und zweiten Signale an die weiteren medizinischen Geräte und/oder Teilnehmer (z. B. Smartphone, zentrales Überwachungsterminal, Pager) weitergeleitet.

Ein weiterer Aspekt der vorliegenden Offenbarung betrifft ein computerimplementiertes Verfahren zur Überwachung eines verteilten Alarmsystems, wobei das verteilte Alarmsystem zumindest einen Server und zumindest ein medizinisches Gerät, die miteinander eine Kommunikationsverbindung eingehen können, umfasst, die Schritte umfassend: Erfassen eines Bestehens einer Kommunikationsverbindung zwischen dem Server und dem medizinischen Gerät; Erhalten von Betriebsdaten für das medizinische Gerät; Erfassen eines Abbrechens der Kommunikationsverbindung zwischen dem Server und dem medizinischen Gerät; Feststellen auf Basis der erhaltenen Betriebsdaten, ob das Abbrechen der Kommunikationsverbindung entweder ein ungewolltes Abbrechen der Kommunikationsverbindung ist oder ein gewolltes Abbrechen der Kommunikationsverbindung ist; Senden eines ersten Signals, wobei das erste Signal ein akustisches Alarmsignal umfasst, wenn ein ungewolltes Abbrechen festgestellt wird;

Senden eines zweiten Signals, wobei das zweite Signal kein akustisches Alarmsignal umfasst, wenn ein gewolltes Abbrechen festgestellt wird.

Gemäß einer bevorzugten weiteren Ausführungsform können die Betriebsdaten auf eine oder mehrere der folgenden Situationen hindeuten: physische Trennung Teilnehmer aus Alarmsystem, Softwareupdate, Wartung Teilnehmer, Wartung Server, Wartung Gateway.

Gemäß einer bevorzugten weiteren Ausführungsform kann der Server ein Gateway zur Konvertierung von Datenprotokollen umfassen.

Gemäß einer bevorzugten weiteren Ausführungsform können das erste und/oder zweite Alarmsignal ein visuelles Alarmsignal umfassen.

Gemäß einer bevorzugten weiteren Ausführungsform kann das Alarmsystem eine Vielzahl an Teilnehmern umfassen.

Gemäß einer bevorzugten weiteren Ausführungsform kann das zumindest eine medizinische Gerät eines oder mehrere der folgenden umfassen: Beatmungsgerät Infusionspumpe, Patientenmonitor.

Gemäß einer bevorzugten weiteren Ausführungsform kann das Verfahren ein automatisches Aufbauen einer erneuten Kommunikationsverbindung zwischen dem zumindest einen medizinischen Gerät und dem Server nach dem erfassten gewollten Abbrechen der Kommunikationsverbindung, insbesondere nach einem Softwareupdate, umfassen.

Weiterhin kann das Verfahren Schritte und Merkmale aufweisen, welche der voranstehend beschriebenen Vorrichtung, insbesondere deren Einrichtungen bzw. Konfigurationen, entsprechen.

Ein weiterer Aspekt der vorliegenden Offenbarung betrifft ein

Computerprogrammprodukt, umfassend Befehle, die bei der Ausführung des Programms durch einen Computer diesen veranlassen, das oben beschriebene Verfahren auszuführen.

Ein weiterer Aspekt der vorliegenden Offenbarung betrifft ein computerlesbares Speichermedium, umfassend Befehle, die bei der Ausführung durch einen Computer diesen veranlassen, das oben beschriebene Verfahren auszuführen.

Ein weiterer Aspekt der vorliegenden Offenbarung betrifft ein medizinisches Überwachungssystem, umfassend: eine oben näher beschriebene Vorrichtung zur Überwachung eines Alarmsystems; einen Server mit einem Gateway; und ein medizinisches Gerät, insbesondere eine Infusionspumpe.

Ein weiterer Aspekt der vorliegenden Offenbarung betrifft eine Verwendung einer Infusionspumpe in einem oben näher beschriebenen medizinischen Überwachungssystem.

Die Einheiten, Vorrichtungen und Geräte gemäß einem oder mehreren Ausführungsbeispielen können unter Verwendung von Hardware, Software und/oder einer Kombination davon implementiert werden. Die Einheiten, Vorrichtungen und Geräte können einteilig oder mehrteilig sein. Hardware-Einheiten, Hardware-Vorrichtung, Hardware-Geräte können beispielsweise durch Verarbeitungsschaltungen wie einen Prozessor, eine Zentraleinheit (CPU), einen Controller, eine arithmetische Logikeinheit (ALU), einen digitalen Signalprozessor, einen Mikrocomputer, ein feldprogrammierbares Gate-Array (FPGA), ein System-on-Chip (SoC), eine programmierbare Logikeinheit, einen Mikroprozessor oder jede andere Vorrichtung, die in der Lage ist, auf Befehle zu reagieren und diese in einer festgelegten Weise auszuführen, implementiert werden.

Die Einheiten, Vorrichtungen und Geräte können eine oder mehrere Schnittstellenschaltungen umfassen. In einigen Beispielen können die Schnittstellenschaltungen verdrahtete oder drahtlose Schnittstellen umfassen, die mit einem lokalen Netz (LAN), dem Internet, einem Weitverkehrsnetz (WAN) oder Kombinationen davon verbunden sind. Die Funktionalität einer bestimmten Einheit, einer Vorrichtung und eines Geräts der vorliegenden Offenbarung können auf mehrere Einheiten, Vorrichtung und Geräte verteilt werden, die über Schnittstellenschaltungen verbunden sind.

Die Einheiten, Vorrichtungen und Geräte gemäß einem oder mehreren Ausführungsbeispielen können auch ein oder mehrere Speichergeräte umfassen. Bei der einen oder den mehreren Speichervorrichtungen kann es sich um materielle oder nichttransitorische computerlesbare Speichermedien handeln, wie Direktzugriffsspeicher (RAM), Festwertspeicher (ROM), ein permanentes Massenspeichergerät (z. B. ein Festplattenlaufwerk), ein Solid-State-Gerät (z. B. NAND Flash) und/oder jeder andere Datenspeichermechanismus, der Daten speichern und aufzeichnen kann. Die eine oder die mehreren Speichervorrichtungen können zum Speichern von Computerprogrammen, Programmcode, Anweisungen oder eine Kombination davon eingerichtet sein.

An dieser Stelle wird explizit darauf hingewiesen, dass auch das medizinische Gerät wie die Infusionspumpe oder das Beatmungsgerät unter die oben genannten Definitionen von Geräte fällt.

Die hier beschriebenen Erläuterungen und Vorteile einzelner Ausführungsformen gelten sinngemäß auch für die anderen Ausführungsformen. Verschiedene beispielhafte Merkmale der Ausführungsformen können erfindungsgemäß kombiniert werden, wo immer dies technisch sinnvoll und machbar ist.

### Kurzbeschreibung der Figuren

Figur 1 zeigt eine schematische Darstellung einer offenbarungsgemäßen Vorrichtung;
Figur 2 zeigt eine schematische Darstellung eines offenbarungsgemäßen medizinischen Überwachungssystems;
Figur 3 zeigt eine schematische Darstellung eines offenbarungsgemäßen Verfahrens;
Figur 4 zeigt ein beispielhaftes Display einer Infusionspumpe bei gewollter abgebrochener Kommunikationsverbindung;
Figur 5 zeigt ein beispielhaftes Display einer Infusionspumpe bei ungewollter abgebrochener Kommunikationsverbindung; und
Figur 6 zeigt ein beispielhaftes Display einer Infusionspumpe bei Bestehen einer Kommunikationsverbindung.

### Detaillierte Beschreibung von bevorzugten Ausführungsformen

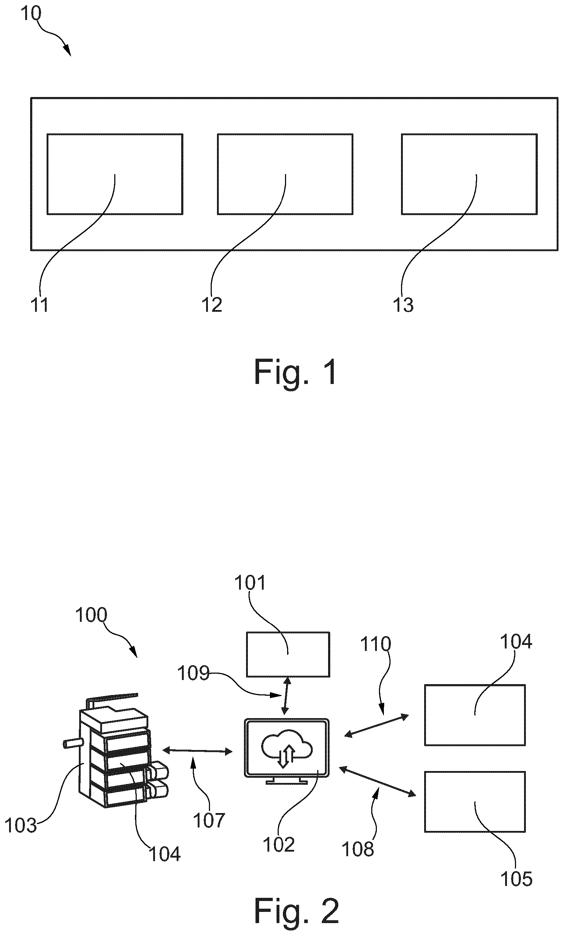

Figur 1 zeigt eine schematische Darstellung einer Vorrichtung 10 zur Überwachung eines verteilten Alarmsystems. Das verteilte Alarmsystem kann dabei beispielsweise zumindest einen Server mit einem Gateway, eine Vielzahl an Infusionspumpen, einen Patientenmonitor sowie eine Vielzahl an mobilen Endgeräten wie Smartphones umfassen. Die Vielzahl an Infusionspumpen und der Patientenmonitor sind dabei jeweils eingerichtet, mit dem Server eine Kommunikationsverbindung eingehen zu können. Die Kommunikationsverbindung erfolgt dabei über ein Funknetzwerk, beispielsweise WLAN oder ein internes Funknetz (DECT) oder eine kabelgebundene Verbindung (Ethernet). Die Vielzahl an Infusionspumpen, der Patientenmonitor, der Server sowie die mobilen Endgeräte haben dafür hardware- und softwareseitig jeweils entsprechende Kommunikationsschnittstellen. Die Vorrichtung 10 umfasst eine Erfassungseinheit 11. Die Erfassungseinheit 11 ist eingerichtet, ein Bestehen einer Kommunikationsverbindung zwischen dem Server und dem medizinischen Gerät zu erfassen. Weiterhin ist die Erfassungseinheit 11 eingerichtet, Betriebsdaten für die Infusionspumpe und den Patientenmonitor zu erfassen. Weiterhin ist die Erfassungseinheit 11 eingerichtet, ein Abbrechen der Kommunikationsverbindung zwischen dem Server und der Vielzahl an Infusionspumpen und dem Patientenmonitor zu erfassen.

Die Vorrichtung 10 umfasst weiter eine Feststellungseinheit 12, die eingerichtet ist, auf Basis der erfassten Betriebsdaten festzustellen, ob das Abbrechen der Kommunikationsverbindung entweder ein ungewolltes Abbrechen der Kommunikationsverbindung ist oder ein gewolltes Abbrechen der

Kommunikationsverbindung ist. Weiterhin umfasst die Vorrichtung 10 eine Sendeeinheit 13, die eingerichtet ist, ein erstes Signal zu senden, wobei das erste Signal ein akustisches Alarmsignal umfasst, wenn ein ungewolltes Abbrechen festgestellt wird. Die Sendeeinheit 13 ist weiter eingerichtet, ein zweites Signal zu senden, wobei das zweite Signal kein akustisches Alarmsignal umfasst, wenn ein gewolltes Abbrechen festgestellt wird. Das zweite Signal umfasst bevorzugt lediglich ein visuelles Signal, das Aufschluss über eine Zustandsänderung der Infusionspumpe gibt. Vorliegend wurde die Infusionspumpe beispielsweise aus einem Ordnungssystem von einem medizinischen Mitarbeiter entnommen. Dabei wurde ein Magnetsensor aktiviert, der ein entsprechendes Signal mittelbar über eine Kommunikationsschnittstelle der Infusionspumpe als Betriebsdaten an die Erfassungseinheit 11 der Vorrichtung gesendet hat. Bei Abbrechen der Kommunikationsverbindung zwischen Infusionspumpe und Server (z. B. weil die Infusionspumpe nun ausgeschaltet wurde) erkennt die Feststellungseinheit 12 durch Auswertung der Betriebsdaten, dass das Abbrechen der Kommunikationsverbindung gewollt ist und sendet lediglich das zweite Signal an die Infusionspumpe, den Patientenmonitor und die mobilen Endgeräte des verteilten Alarmsystems. Wenn beispielsweise jedoch durch die Auswertung der Betriebsdaten von der Feststellungseinheit 12 festgestellt wird, dass die Kommunikationsverbindung plötzlich etwa während einer laufenden Behandlung abgebrochen ist und somit das Abbrechen der Kommunikationsverbindung ungewollt ist, sendet die Feststellungseinheit 12 das erste Signal, welches das akustische Alarmsignal umfasst, an die Infusionspumpe, den Patientenmonitor und die mobilen Endgeräte des verteilten Alarmsystems.

Fig. 2 zeigt schematisch ein medizinisches Überwachungssystem 100. Das medizinische Überwachungssystem 100 umfasst eine in Fig. 1 näher beschriebene Vorrichtung 101 zur Überwachung eines verteilten Alarmsystems. Das medizinische Überwachungssystem 100 umfasst weiter einen Server mit Gateway 102 sowie eine Infusionspumpe 104, die in einem Ordnungssystem 103 angeordnet ist. Weiterhin sind vorliegend ein Patientenmonitor 104 und ein weiteres medizinisches Gerät 105, beispielsweise ein Beatmungsgerät Teil des medizinischen Überwachungssystems 100. Die Infusionspumpe 104 steht über eine Kommunikationsverbindung 107 mit dem Server 102 in Verbindung. Die Vorrichtung 101 steht über eine

Kommunikationsverbindung 109 mit dem Server 102 in Verbindung. Alternativ könnte die Vorrichtung 101 auch Teil des Servers 102 sein. Alternativ könnte die Vorrichtung 101 auch Teil des medizinischen Geräts 105 oder der Infusionspumpe 104 sein. Das medizinische Gerät 105 steht über eine Kommunikationsverbindung 108 mit dem Server 102 in Verbindung. Die Infusionspumpe 104 steht über eine Kommunikationsverbindung 107 mit dem Server 102 in Verbindung. Die Kommunikationsverbindungen sind vorliegend funkbasiert, zum Beispiel über WLAN. Alternativ oder ergänzend können die Kommunikationsverbindungen auch kabelgebunden sein. Beispielsweise kann die Anbindung zwischen Server/Gateway und Patientenmonitor und/oder zwischen Server/Gateway und Beatmungsgerät kabelgebunden sein. Die kabelgebundene Kommunikationsverbindung kann über Ethernet erfolgen. Über die Kommunikationsverbindungen können vorliegend Betriebsdaten sowie erste Signale und zweite Signale ausgetauscht werden. Beispielsweise empfängt die Vorrichtung 101 die Information, dass die Infusionspumpe 104 aus dem Ordnungssystem entnommen wird als Betriebsdaten. Im Falle eines Abbruchs der Kommunikationsverbindung 107, sendet die Vorrichtung 101 dann lediglich ein zweites Signal an das medizinische Gerät 105 und die Infusionspumpe 104, da sie ein gewolltes Abbrechen erkannt hat.

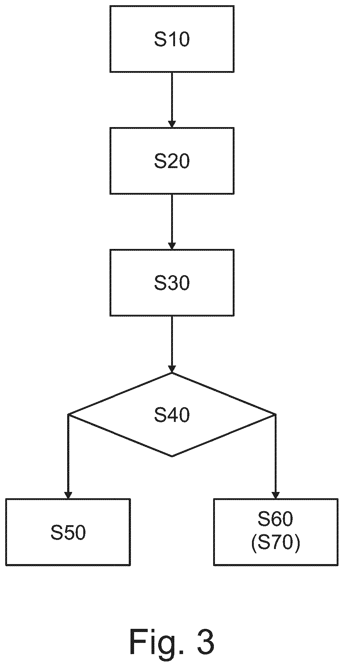

Fig. 3 zeigt eine schematische Darstellung eines computerimplementierten Verfahrens zur Überwachung eines medizinischen Überwachungssystems. Das medizinische Überwachungssystem umfasst dabei zumindest einen Server mit Gateway und zumindest ein medizinisches Gerät, die miteinander eine Kommunikationsverbindung eingehen können. Das Verfahren umfasst folgende Schritte.

Schritt S10 umfasst ein Erfassen eines Bestehens einer Kommunikationsverbindung zwischen dem Server und dem medizinischen Gerät. Hierzu wertet eine Erfassungseinheit einen Heart Beat des medizinischen Geräts aus. Bei Vorliegen eines entsprechenden Signals liegt eine Kommunikationsverbindung vor.

Schritt S20 umfasst ein Erfassen von Betriebsdaten für das medizinische Gerät. Hierzu empfängt die Erfassungseinheit über die Kommunikationsverbindung Betriebsdaten über das medizinische Gerät, beispielsweise Informationen zu einem anstehenden Softwareupdate.

Schritt S30 umfasst ein Erfassen eines Abbrechens der Kommunikationsverbindung zwischen dem Server und dem medizinischen Gerät. Hierzu werte die Erfassungseinheit den Heart Beat aus. Bei Ausbleiben eines entsprechenden Signals, wird ein Abbrechen einer Kommunikationsverbindung angenommen.

Schritt S40 umfasst ein Feststellen auf Basis der erhaltenen Betriebsdaten, ob das Abbrechen der Kommunikationsverbindung entweder ein ungewolltes Abbrechen der Kommunikationsverbindung ist oder ein gewolltes Abbrechen der Kommunikationsverbindung ist. Hierzu vergleicht eine Feststellungseinheit, ob die Betriebsdaten auf ein gewolltes Abbrechen der Kommunikationsverbindung hindeuten. Vorliegend führt das Softwareupdate zu einer temporären Unterbrechung der Kommunikationsverbindung. Die Feststellungseinheit erkennt dies, da sie im Vorfeld in Schritt S20 entsprechende Betriebsdaten erhalten hat. Somit liegt als Ergebnis ein festgestelltes gewolltes Abbrechen der Kommunikationsverbindung vor.

Schritt S50 umfasst ein Senden eines ersten Signals, wobei das erste Signal ein akustisches Alarmsignal umfasst, wenn ein ungewolltes Abbrechen festgestellt wird. Vorliegend wird kein erstes Signal gesendet, da ein gewolltes Abbrechen festgestellt wurde.

Schritt S60 umfasst ein Senden eines zweiten Signals, wobei das zweite Alarmsignal kein akustisches Alarmsignal umfasst, wenn ein gewolltes Abbrechen festgestellt wird. Vorliegend wird durch eine Sendeeinheit ein zweites Signal an beispielsweise mobile Endgeräte von medizinischen Mitarbeitern gesendet, da ein gewolltes Abbrechen festgestellt wurde. Gemäß einem vorteilhaften Aspekt kann der Schritt S60 weiterhin umfassen bzw. kann nach dem Schritt S60 ein weiterer Schritt S70 folgen, nämlich automatisches Aufbauen einer erneuten Kommunikationsverbindung 107 zwischen der Infusionspumpe 104 und dem Server 102 nach dem Softwareupdate

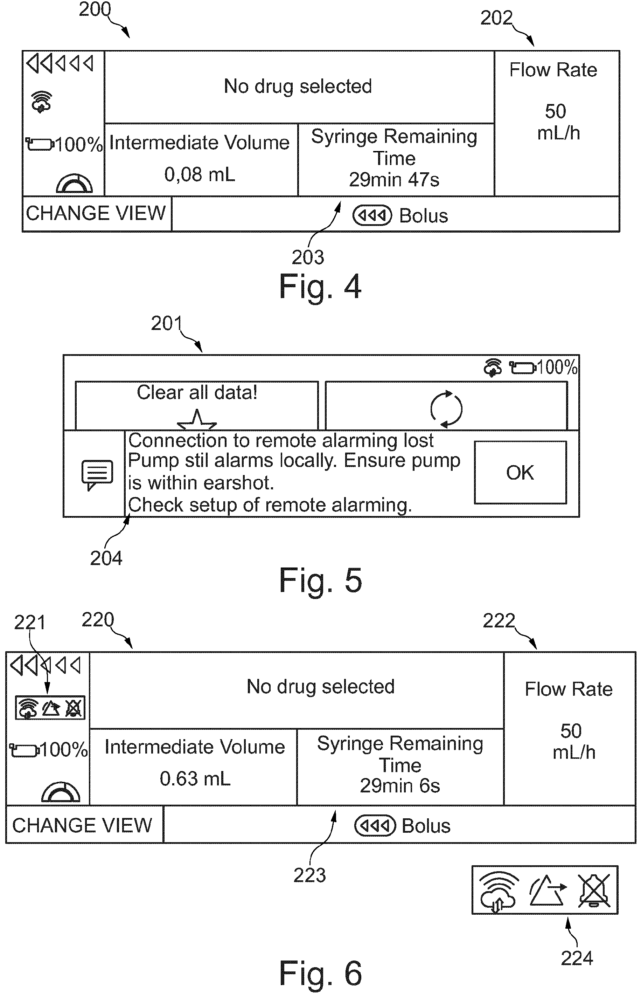

Figur 4 zeigt ein beispielhaftes Display 201 einer Infusionspumpe bei gewollter abgebrochener Kommunikationsverbindung. Mit anderen Worten ausgedrückt, ist die Infusionspumpe keinem verteilten Alarmsystem zugeordnet. In diesem Zustand werden unterschiedliche Werte wie Flussrate 202 und verbleibende Infusionsdauer 203 an dem Display 201 angezeigt. Es wurde kein akustischer Alarm beim Austreten aus dem verteilten Alarmsystem abgegeben.

Fig. 5 zeigt ein beispielhaftes Display 201 in einem Zustand bei ungewollter abgebrochener Kommunikationsverbindung. In dem Zustand bei ungewollt abgebrochener Kommunikationsverbindung erscheint auf dem Display 201 eine Warnung 204, dass die Kommunikationsverbindung zum verteilten Alarmsystem abgebrochen ist. Weiterhin gibt die Infusionspumpe einen akustischen Alarm zusätzlich zu der Warnung 204 aus.

Figur 6 zeigt ein beispielhaftes Display 220 einer Infusionspumpe bei Bestehen einer Kommunikationsverbindung. Hierbei werden die normalen Werte wie Flussrate 222 und verbleibende Infusionsdauer 223 weiterhin angezeigt. Es wird weiterhin angezeigt, dass das akustisches Alarmsignal 224 an der Infusionspumpe stummgeschalten ist. Dies wird im Bereich 221 angezeigt. In anderen Worten teilt die Anzeige im Bereich 221 einem Anwender mit, dass die Kommunikationsverbindung zwischen der Infusionspumpe und dem Server besteht, dass die Infusionspumpe im verteilten Alarmsystem eingeloggt ist und bei Auftreten einer Gefahrensituation, ein akustischer Alarm nicht an der Infusionspumpe auftritt sondern beispielsweise an einem mobilen Endgerät eines Pflegers.

### Bezugszeichenliste

- 10: Vorrichtung
- 11: Erfassungseinheit
- 12: Feststellungseinheit
- 13: Sendeeinheit

- 100: Medizinisches Überwachungssystem
- 101: Vorrichtung
- 102: Server mit Gateway
- 103: Ordnungssystem
- 104: Infusionspumpe
- 105: medizinisches Gerät
- 106: Patientenmonitor
- 107, 108, 109, 110: Kommunikationsverbindung
- 200,201,220: Display
- 202, 222: Flussrate
- 203, 223: Infusionsdauer
- 204: Warnung
- 221: Bereich Display
- 224: ausgegrautes akustisches Alarmsignal

- S10: Erfassen eines Bestehens einer Kommunikationsverbindung
- S20: Erfassen von Betriebsdaten für das medizinische Gerät
- S30: Erfassen eines Abbrechens der Kommunikationsverbindung
- S40: Feststellen ungewolltes Abbrechen oder gewolltes Abbrechen der Kommunikationsverbindung auf Basis der Betriebsdaten
- S50: Senden eines ersten Signals bei ungewolltem Abbrechen
- S60: Senden eines zweiten Signals bei gewolltem Abbrechen
- S70: Automatisches Wiederaufbauen einer Kommunikationsverbindung zwischen medizinischem Gerät und Server nach dem Softwareupdate

## Patentansprüche

1. Vorrichtung (10, 101) zur Überwachung eines verteilten Alarmsystems, wobei das verteilte Alarmsystem zumindest einen Server (102) und zumindest ein medizinisches Gerät (104, 105), die miteinander eine Kommunikationsverbindung (107, 108, 110) eingehen können, umfasst, umfassend:
eine Erfassungseinheit (11), die eingerichtet ist, ein Bestehen einer Kommunikationsverbindung (107, 108, 110) zwischen dem Server (102) und dem medizinischen Gerät (104, 105) zu erfassen;
wobei die Erfassungseinheit (11) weiter eingerichtet ist, Betriebsdaten für das medizinische Gerät (104, 105) zu erfassen;
wobei die Erfassungseinheit (11) weiter eingerichtet ist, ein Abbrechen der Kommunikationsverbindung (107, 108, 110) zwischen dem Server (102) und dem medizinischen Gerät (104, 105) zu erfassen;
eine Feststellungseinheit (12), die eingerichtet ist, auf Basis der erfassten Betriebsdaten festzustellen, ob das Abbrechen der Kommunikationsverbindung (107, 108, 110) entweder ein ungewolltes Abbrechen der Kommunikationsverbindung (107, 108, 110) ist oder ein gewolltes Abbrechen der Kommunikationsverbindung (107, 108, 110) ist;
eine Sendeeinheit (13), die eingerichtet ist, ein erstes Signal zu senden, wobei das erste Signal ein akustisches Alarmsignal umfasst, wenn ein ungewolltes Abbrechen festgestellt wird;
wobei die Sendeeinheit (13) weiter eingerichtet ist, ein zweites Signal zu senden, wobei das zweite Signal kein akustisches Alarmsignal umfasst, wenn ein gewolltes Abbrechen festgestellt wird.

2. Vorrichtung (10, 101) nach Anspruch 1, wobei die Betriebsdaten auf eine oder mehrere der folgenden Situationen hindeuten: physische Trennung Teilnehmer aus Alarmsystem, Softwareupdate, Wartung Teilnehmer, Wartung Server (102), Wartung Gateway.

3. Vorrichtung (10, 101) nach Anspruch 1 oder 2, wobei der Server (102) ein Gateway zur Konvertierung von Datenprotokollen umfasst.

4. Vorrichtung (10, 101) nach einem der vorherigen Ansprüche, wobei das erste und/oder zweite Signal ein visuelles Signal umfasst.

5. Vorrichtung (10, 101) nach einem der vorherigen Ansprüche, wobei das Alarmsystem eine Vielzahl an medizinischen Geräten (104, 105) und/oder anderen Teilnehmern umfasst.

6. Vorrichtung (10, 101) nach einem der vorherigen Ansprüche, wobei das medizinische Gerät (104, 105) eine Infusionspumpe (104) ist.

7. Vorrichtung (10, 101) nach einem der vorherigen Ansprüche, weiter umfassend automatisches Aufbauen einer erneuten Kommunikationsverbindung (107, 108, 110) zwischen dem medizinischen Gerät (104, 105) und dem Server (102) nach dem erfassten gewollten Abbrechen der Kommunikationsverbindung (107, 108, 110), insbesondere nach einem Softwareupdate.

8. Vorrichtung (10, 101) nach einem der vorherigen Ansprüche, wobei das erste und/oder zweite Signal an den Server (102) zur Weiterleitung an weitere medizinische Geräte (104, 105) und/oder Teilnehmer versendet wird.

9. Computerimplementiertes Verfahren zur Überwachung eines verteilten Alarmsystems, wobei das verteilte Alarmsystem zumindest einen Server (102) und zumindest ein medizinisches Gerät (104, 105), die miteinander eine Kommunikationsverbindung (107, 108, 110) eingehen können, umfasst, wobei das Verfahren die Schritte umfasst:
Erfassen eines Bestehens einer Kommunikationsverbindung (107, 108, 110) zwischen dem Server (102) und dem medizinischen Gerät (104, 105) (S10);
Erfassen von Betriebsdaten für das medizinische Gerät (104, 105) (S20);
Erfassen eines Abbrechens der Kommunikationsverbindung (107, 108, 110) zwischen dem Server (102) und dem medizinischen Gerät (104, 105) (S30);
Feststellen auf Basis der erhaltenen Betriebsdaten, ob das Abbrechen der Kommunikationsverbindung (107, 108, 110) entweder ein ungewolltes Abbrechen der Kommunikationsverbindung (107, 108, 110) ist oder ein gewolltes Abbrechen der Kommunikationsverbindung (107, 108, 110) ist (S40);
Senden eines ersten Signals, wobei das erste Signal ein akustisches Alarmsignal umfasst, wenn ein ungewolltes Abbrechen festgestellt wird (S50);
Senden eines zweiten Signals, wobei das zweite Signal kein akustisches Alarmsignal umfasst, wenn ein gewolltes Abbrechen festgestellt wird (S60).

10. Verfahren nach Anspruch 9, wobei die Betriebsdaten auf eine oder mehrere der folgenden Situationen hindeuten: physische Trennung Teilnehmer aus Alarmsystem, Softwareupdate, Wartung Teilnehmer, Wartung Server (102), Wartung Gateway.

11. Verfahren nach Anspruch 9 oder 10, wobei der Server (102) ein Gateway zur Konvertierung von Datenprotokollen umfasst.

12. Computerprogrammprodukt, umfassend Befehle, die bei der Ausführung des Programms durch einen Computer diesen veranlassen, das Verfahren nach einem der Ansprüche 9 bis 11 auszuführen.

13. Computerlesbares Speichermedium, umfassend Befehle, die bei der Ausführung durch einen Computer diesen veranlassen, das Verfahren nach einem der Ansprüche 9 bis 11 auszuführen.

14. Medizinisches Überwachungssystem, umfassend:
eine Vorrichtung (10, 101) zur Überwachung eines verteilten Alarmsystems nach einem der Ansprüche 1 bis 8;
einen Server (102) mit einem Gateway;
mindestens ein medizinisches Gerät (104, 105), insbesondere mindestens eine Infusionspumpe (104).

15. Verwendung einer Infusionspumpe in einem Überwachungssystem nach Anspruch 14.
